# EUROPEAN PATENT APPLICATION

(11) **EP 3 208 264 A1**
(43) Date of publication of application: **23.08.2017**
(21) Application number: 16156505.6
(22) Date of filing: 19.02.2016
(51) Int. Cl.: C07D 251/60, C07C 273/12

(54) **METHOD FOR REVAMPING A HIGH PRESSURE MELAMINE PLANT**

(71) Applicant: Casale SA, 6900 Lugano (CH)
(72) Inventor: DI CARLO, Gabriele, 6900 Lugano (CH); SCOTTO, Andrea, 6932 Breganzona (CH); GAMBA, Simone, 24040 Pagazzano (BG) (IT)
(74) Representative: Zardi, Marco

(57) **Abstract**

A high pressure melamine plant comprising a synthesis section and a melamine purification section, wherein the synthesis section provides a first melamine-containing stream (6), and said melamine purification section comprises: a quencher (2) receiving said first melamine-containing stream (6) from the synthesis section; a stripper (3) fed with a second melamine-containing stream (7) from said quencher (2), wherein purified melamine (11) is collected and vapours (10) are extracted; a heat exchanger (12), wherein said vapours (10) are condensed providing a condensed stream (14); an absorber (4) fed with said condensed stream (14) providing an aqueous solution (15) comprising ammonia and carbon dioxide, at least a portion (15b) of said aqueous solution being exported from the purification section (200) of the plant.

## Description

### Field of application

The invention relates to the field of melamine production from urea and relates in particular to the purification section of a high pressure melamine plant, a related process and a method of revamping.

### Prior Art

The processes for the synthesis of melamine from urea comprise low pressure catalytic processes and high pressure non-catalytic processes, typically above 7 MPa. These processes are well-known in the art.

A conventional type of plant for the synthesis of melamine using the high pressure non-catalytic process comprises a high pressure section substantially limited to a reactor, called melamine reactor, wherein the urea melt feed is converted into raw melamine with addition of heat.

Conversion of urea into melamine also generates gases mainly consisting of ammonia and carbon dioxide (so-called "off-gas") and a number of by-products (mainly OATs and polycondensates). The reaction products (melamine and off-gas), the by-products and the unreacted urea are further processed in a purification section operating at significantly lower pressure and temperature and generally comprising a quencher, a stripper and an absorber.

In the quencher, raw melamine is dissolved in an ammonia aqueous solution to separate off-gas from melamine. The off-gas are conveyed to a condensation section and melamine is supplied to the stripper, preferably a steam stripper, for removal of residual off-gas. The latter are absorbed in water inside the absorber and melamine is subjected to further purification in downstream equipment.

This configuration is widely used, but has a number of drawbacks.

Generally, the melamine plant is combined with a urea plant which produces the urea melt feed, starting from NH₃ and CO₂. The off-gas extracted from the quencher of the melamine plant are thus generally recycled to the combined urea plant. However, this implies condensation of the off-gas into a solution containing NH₃ and CO₂ suitable to be conveyed back to the urea plant, and a related condensation section.

Moreover, in order to avoid ammonia losses from the absorber and minimize the carbon dioxide content in the melamine solution leaving the stripper, a sequence of desorption and absorption occurring, respectively, in the stripper and the subsequent absorber is strongly required. However, it implies a high steam consumption inside the stripper, which is very disadvantageous in terms of costs.

### Summary of the invention

The purpose of the invention is to avoid the above drawbacks of the prior art.

Said purpose is achieved with a high pressure melamine plant, a related process and a method of revamping according to the claims.

The term "high pressure melamine plant" denotes a plant for the synthesis of melamine using a high pressure non-catalytic process, which comprises a synthesis section operating at a high pressure, which is typically greater than 7 MPa, and a purification section operating at a lower pressure, which is typically 2 to 3 MPa.

The high pressure melamine plant according to the invention comprises a synthesis section and a melamine purification section, wherein said synthesis section provides a first melamine-containing stream, and said purification section comprises:
a quencher receiving said first melamine-containing stream from the synthesis section;
a stripper fed with a second melamine-containing stream from said quencher and with a stripping medium, said stripping medium being preferably steam, wherein purified melamine is obtained and vapours are extracted;
a heat exchanger, wherein said vapours are at least partially condensed by heat exchange with a cooling medium, obtaining a condensed stream;
an absorber fed with said condensed stream from said heat exchanger and providing an aqueous solution comprising ammonia and carbon dioxide, at least a portion of said aqueous solution being exported from the purification section of the plant. Preferably, said aqueous solution is at least partially recycled to a tank.

Preferably, the vapours extracted from the stripper contain quantities of ammonia and carbon dioxide.

The term "first melamine-containing stream" refers to a raw melamine melt and the term "second melamine-containing stream" refers to a melamine solution.

Advantageously, a first portion of the aqueous solution extracted from the absorber is recirculated inside the absorber itself, where it acts as absorbing medium for said condensed stream, and a second portion of said aqueous solution is exported from the purification section of the plant. More advantageously, said first portion is recirculated inside said absorber after passing through a cooler.

Preferably, a line is arranged to introduce a stream of water into the purification section of the melamine plant, a first portion of said stream of water forming said cooling medium and a second portion thereof bypassing the heat exchanger and being supplied to the quencher.

Preferably, said stream of water is a recycle stream coming from the plant.

According to an advantageous embodiment of the invention, said first and second portions of the stream of water contain ammonia. Ammonia is advantageously added to said stream of water before the splitting into the above first and second portions.

Addition of ammonia to said stream of water is particularly preferred because ammonia is able to maintain an adequate pH value in the quencher and prevents the formation of oxi amino triazines (OATs).

According to preferred embodiments, the portion of water leaving the above heat exchanger is at least partially sent back to the quencher. According to even more preferred embodiments, said portion of water leaving the above heat exchanger is at least partially heated in a further heat exchanger and then supplied to the quencher.

According to a particularly preferred embodiment, the synthesis section of the high pressure melamine plant comprises a melamine reactor, a stripping reactor and a scrubber.

Preferably, the scrubber is fed with at least part of the urea melt directed to the high pressure synthesis section. In the scrubber, the urea melt is preheated by using off-gas supplied by the melamine and/or stripping reactor as heat source. The melamine reactor is at least partially fed with the urea melt leaving the scrubber, providing raw melamine and separating off-gas. The second stripping reactor is fed with said raw melamine and gaseous ammonia as stripping agent, providing purified melamine and separating off-gas. At least a portion of the off-gas extracted from said melamine reactor and/or from said stripping reactor are fed to the scrubber, inside which the off-gas are brought into contact with the urea melt feed.

Even more preferably, said synthesis section also comprises a steam generator, which is supplied with a portion of the urea melt leaving the scrubber as heat source.

The term "purified melamine" refers to the melamine melt obtained in the stripping reactor, which has higher purity than the raw melamine obtained in the melamine reactor, but which needs be further purified in the subsequent purification section.

The term "high pressure section" denotes that the melamine reactor, the stripping reactor and the scrubber operate substantially at the same nominal pressure, said pressure being greater than 7 MPa and preferably greater than 10 MPa, for example 11 MPa.

Another aspect of the present invention concerns the revamping of the purification section of a prior art melamine plant comprising:
a quencher fed with a first melamine-containing stream collected from the synthesis section of the plant and an aqueous solution comprising ammonia and carbon dioxide, wherein melamine is dissolved and off-gas are extracted;
a stripper fed with a second melamine-containing stream from said quencher and with a stripping medium, said stripping medium being preferably steam, wherein purified melamine is obtained and vapours are extracted, said vapours being added with a stream of water once extracted from the stripper;
a first heat exchanger, wherein the vapours added with said stream of water are at least partially condensed by heat exchange with an aqueous solution comprising ammonia and carbon dioxide, providing a condensed stream;
an absorber fed with said condensed stream and a carbonate solution, which provides an aqueous solution comprising ammonia and carbon dioxide, said aqueous solution being at least partially used for condensing the vapours in said first heat exchanger, further heated in a second heat-exchanger and recycled to the quencher. Said carbonate solution is preferably recycled from an ammonia separation section.

Said method of revamping is characterized by: installation of a line for at least partially feeding a stream of water (possibly added with ammonia) to the first heat exchanger for condensing said vapours; installation of a line for at least partially sending the stream of water leaving the first heat exchanger to the quencher; installation of a line for at least partially exporting the aqueous solution provided by said absorber from the purification section of the plant; discontinuing the line for feeding said aqueous solution to the first heat exchanger, for subsequent feeding to the second exchanger and recycle to the quencher; discontinuing the line for adding water to the vapours extracted from the stripper; discontinuing the line for introducing the carbonate solution into the absorber.

Preferably, said stream of water at least partially supplied to the first heat exchanger is a recycle stream coming from the plant. More preferably, a line is installed for injecting ammonia into said stream of water.

According to a preferred embodiment, a line bypassing the first heat exchanger is installed for supplying a portion of said stream of water, possibly added with ammonia, to the quencher. Even more preferably, said line is installed downstream of the line for injecting ammonia into said stream of water, the ammonia being able to maintain an adequate pH value in the quencher preventing the formation of OATs.

Advantageously, the line of water leaving the first heat exchanger passes through said second heat exchanger for further heating and then is supplied to the quencher.

According to an embodiment of the prior art, a further portion of the aqueous solution extracted from the absorber is directly supplied to the quencher and, according to the method of the invention, said further line is discontinued.

Advantageously, the line for extracting the off-gas from the quencher is discontinued due to the lack of carbon dioxide and ammonia leaving the quencher as gaseous stream.

According to another embodiment of the invention, the above described purification section of the prior art is revamped by: installing a line feeding a stream of water to the quencher, at least a portion of said stream of water being heated inside said second heat exchanger before being sent to the quencher; dismissing the stripper; dismissing the first heat exchanger; redirecting the stripping medium to the quencher; installing a line for exporting said second melamine-containing stream from the purification section of the plant; dismissing the absorber. Preferably, a line is installed for injecting ammonia into said stream of water.

Said second melamine-containing stream from the quencher is preferably subjected to further purification treatment downstream said purification section.

More preferably, a pump is installed downstream of the quencher for suitably pressurizing said second melamine-containing stream before being subjected to said further purification.

According to particularly advantageous embodiments of the invention, the above methods of revamping concerns the purification section of a high pressure melamine plant of the prior art provided with a synthesis section comprising a melamine reactor, a stripping reactor and a scrubber, as disclosed in the patent application No. EP15154205.7.

The present invention has the following advantages.

First, the undesired sequence of desorption and absorption taking place, respectively, in the stripper and the absorber is avoided because the ammonia content in the absorber is minimized, as well as the carbon dioxide content in the effluent from the stripper. As a consequence, the steam consumption inside the stripper is significantly reduced or eliminated in the case the revamping provides for the stripper elimination.

A further advantage is that the liquid stream leaving the quencher is not saturated with ammonia and carbon dioxide, which provides for less off-gas extracted from the subsequent stripping and a lower content of ammonia and carbon dioxide in the aqueous solution collected from the absorber. An even lower content of ammonia and carbon dioxide in the liquid stream leaving the quencher justifies the dismissing of the stripper and the absorber.

Moreover, the quencher does not provide any gaseous stream and no off-gas are recovered therefrom. As a consequence, an off-gas condensation unit is no longer required.

Another advantage is that a portion of the aqueous solution from the absorber is exported from the purification section for further use in the process, thanks to the fact that the water input for the quencher is provided by a stream of water recycled from the plant.

The advantages will emerge even more clearly with the aid of the detailed description below relating to preferred embodiments.

### Description of the figures

Fig. 1 is a block scheme of the melamine plant according to a preferred embodiment of the invention.
Fig. 2 is a schematic diagram of the high-pressure section of the plant according to Fig. 1.
Fig. 3 is a block scheme of a melamine plant according to the prior art.
Fig. 4 is a block scheme of a melamine plant after revamping of the plant of Fig. 3, according to another embodiment of the invention.

### Detailed description

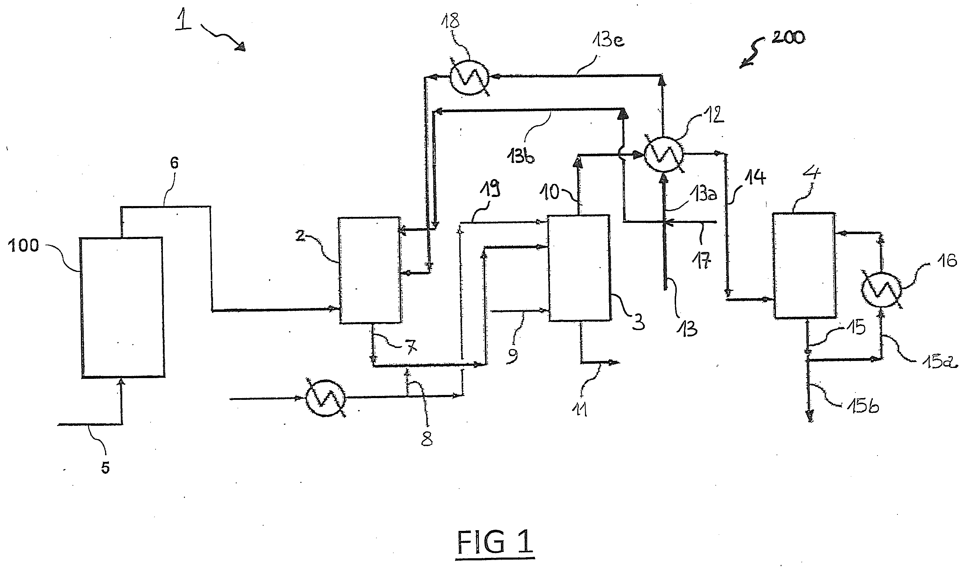

With reference to Fig. 1, the melamine plant 1 comprises a high-pressure section 100 and a purification section 200 which operates at significantly lower pressure and temperature.

The high-pressure section 100 is fed with urea melt 5 and produces melamine 6. As the melamine 6 leaves the high-pressure section 100, the pressure is lowered from above 7 MPa to 0.4-2 MPa and melamine is supplied to the purification section 200.

Said purification section 200 essentially comprises a quencher 2, a stripper 3 and an absorber 4. In the quencher 2, melamine 6 is treated at around 160 °C with aqueous solutions of ammonia 13c, 13b to dissolve melamine. Melamine is collected from the bottom of the quencher 2 as an aqueous solution 7, further diluted with a stream of water 8 coming from the plant and fed to the stripper 3.

The stripper 3 is further fed with steam 9, which preferably comes from the plant, and with a further stream of water 19 coming from the plant. In the stripper 3, vapours 10 are extracted from the top and an ammonia- and carbon dioxide- free melamine solution 11 is collected from the bottom. Said solution 11 is then subjected to further purification, comprising for example: filtration, clarification with activated carbon, crystallization, solid separation of the melamine crystals and drying.

The vapours 10 are then sent to a heat exchanger 12, where they are condensed by thermal exchange with an aqueous solution of ammonia 13a.

The condensed vapours 14 are fed into the absorber 4, which provides an aqueous solution 15. Said aqueous solution is split into two portions: the first portion 15a is passed through a cooler 16 and recirculated inside the absorber 4 and the second portion 15b is exported from the purification section 200 and preferably recycled to the plant.

A stream of water 13 recycled from the plant and an ammonia stream 17 are mixed to provide an aqueous ammonia solution, which is split into portions 13a, 13b. The first portion 13a is fed to the above heat exchanger 12, where it is preheated to provide stream 13c, and the second portion 13b is supplied to the quencher 2.

Said stream 13c of preheated aqueous solution is fed to another heat exchanger 18, where it is further heated, and supplied to the quencher 2.

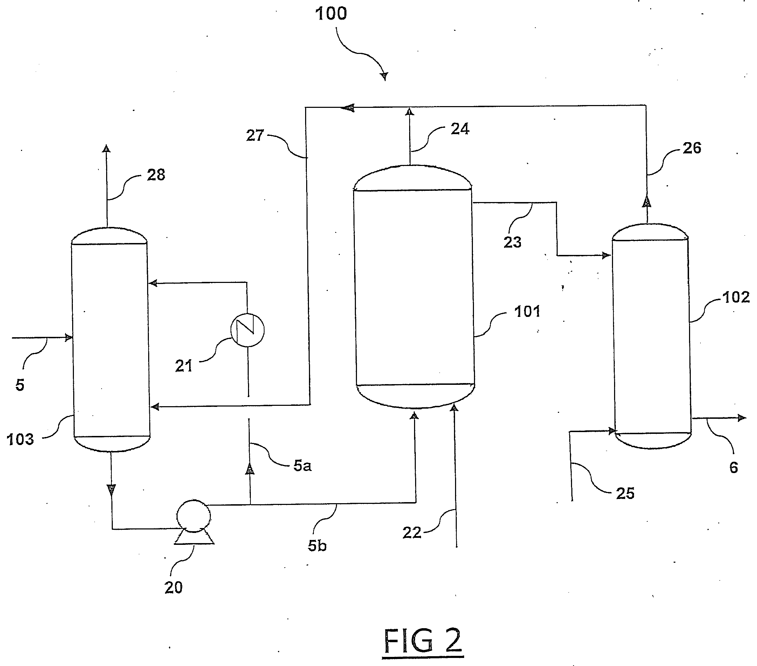

Fig. 2 shows the high-pressure section 100 of the plant 1 of Fig. 1 according to an embodiment of the invention, which comprises essentially a first melamine reactor 101, a second stripping reactor 102 and a scrubber 103.

In particular, the scrubber 103 is fed with the urea melt 5. The urea melt leaving the scrubber passes through a pump 20 and a first portion 5a of said urea melt is recirculated inside the scrubber 103 after passing through a heat exchanger 21, where it is advantageously cooled producing vapour, and a second portion 5b of said urea melt is supplied to the melamine reactor 101.

The melamine reactor 101 is also fed with a heat-carrier fluid 22 which supplies heat to the melamine reactor 101 in order to promote the endothermic reaction for conversion of urea into melamine. Typically said heat-carrier fluid 22 consists of molten salts which circulate inside suitable heating pipes of the reactor.

Raw melamine is separated as stream 23 and off-gas are extracted as stream 24.

Said raw melamine 23 is sent to the second stripping reactor 102, which is further fed with gaseous ammonia 25 as stripping agent. The purified melamine 6 leaving the stripping reactor 102 is fed to the quencher 2 of the purification section 200 of the plant, generally operating at a lower pressure than the synthesis section 100, and the off-gas 26 extracted therefrom are combined with the off-gas 24 from the melamine reactor 101, providing a stream 27.

Said stream 27 is introduced into the scrubber 103, where it undergoes washing with the urea melt streams 5 and 5a, respectively feeding and recirculating streams. A high pressure stream of anhydrous off-gas 28, mainly composed of ammonia and carbon dioxide, is extracted from the scrubber 103.

For example, the urea melt 5 is produced in a urea plant connected to the melamine plant comprising the section 100. The off-gas 28, in view of their content of NH3 and CO2 (which constitute the reagents for obtaining urea), are preferably recycled to said urea plant.

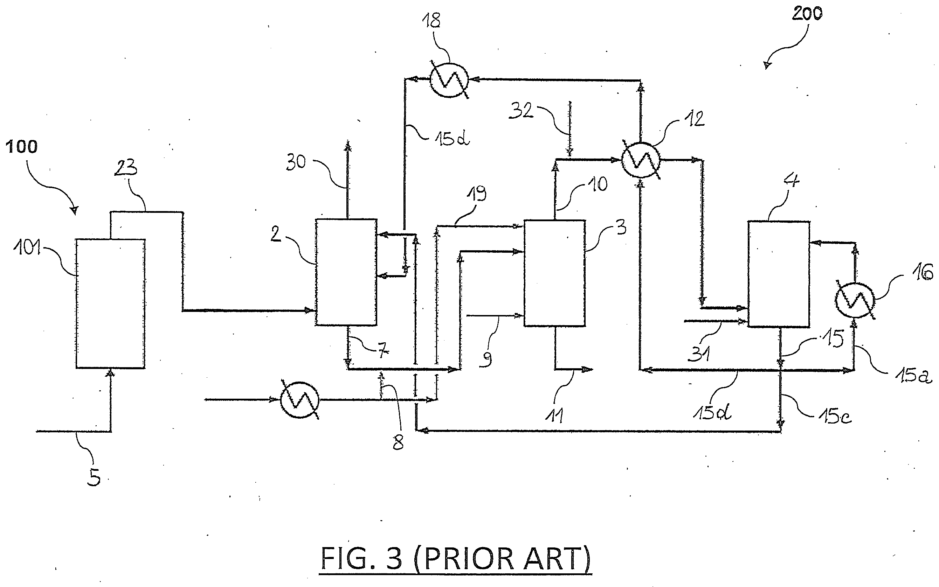

Fig. 3 shows a melamine plant according to the prior art, comprising a high-pressure section 100 and a purification section 200.

The high-pressure section 100 essentially comprises the melamine reactor 101, which is fed with the urea melt 5 and produces a stream 23 containing melamine and off-gas, which is supplied to the purification section 200.

The purification section 200 essentially comprises a quencher 2, a stripper 3 and an absorber 4. In the quencher 2, which is fed with said stream 23 containing melamine and off-gas and an aqueous solution 15d comprising ammonia and carbon dioxide, melamine 7 is collected from the bottom and off-gas 30 are extracted from the top.

The off-gas 30 leaving the top of the quencher 2 are saturated with water and conveyed to a condensation section (not shown); melamine 7 is diluted with a stream of water 8 and fed to the stripper 3, which is supplied with steam 9 and a further stream of water 19.

Vapours 10, which contain ammonia and carbon dioxide, are removed from the top of the stripper 3 and an ammonia- and carbon dioxide- free melamine solution 11 is collected from the bottom thereof. Said solution 11 is then subjected to further purification.

The vapours 10 are added with an amount of water 32, preferably recycled from the plant, condensed in a heat exchanger 12 and then absorbed in water within the absorber 4.

Said absorber is also fed with a carbonate solution 31, providing an aqueous solution 15. Said aqueous solution 15 is split into three portions: the first portion 15a is passed through a cooler 16 and recirculated inside the absorber 4, the second portion is recycled to the quencher 2 through a first line 15c and the third portion is recycled to the quencher 2 through a second line 15d.

The first line 15c provides direct supply of the above solution to the quencher 2, while the second line 15d provides: passage of the solution through the above mentioned heat exchanger 12 for condensing the off-gas 10 extracted from the stripper 3; passage through a further heat exchanger 18 where it is further heated; supply to the quencher 2.

Said plant of the prior art is advantageously revamped to provide the melamine plant illustrated in Fig. 1, by means of the following operations:
installation of a line 13 for recycling a stream of water from the plant;
installation of a line 17 for injecting ammonia into said stream of water;
installation of a line 13a for feeding a first portion of said stream of water containing ammonia to the first heat exchanger 12, wherein said stream of water 13a is preheated;
installation of a line 13b for feeding a second portion of said stream of water containing ammonia to the quencher 2;
installation of a line 13c for sending the preheated water to the second heat exchanger 18 and then to the quencher 2;
installation of a line 15b for at least partially exporting the aqueous solution
provided by said absorber 4 from the purification section 200 of the plant;
discontinuing the line 15d for feeding said aqueous solution to the first heat exchanger 12, for subsequent feeding to the second-exchanger 18 and recycle to the quencher 2;
discontinuing the line 15c for directly supplying said aqueous solution to the quencher 2;
discontinuing the line 32 for adding said stream of water 32 to the vapours 10 extracted from the stripper 3;
discontinuing the line 31 for introducing the carbonate solution into the absorber 4;
discontinuing the line 30 for extracting the off-gas 30 from the quencher 2.

According to this embodiment, the revamping of the purification section 200 is carried out after revamping of the high pressure synthesis section to provide the synthesis section of Fig. 2.

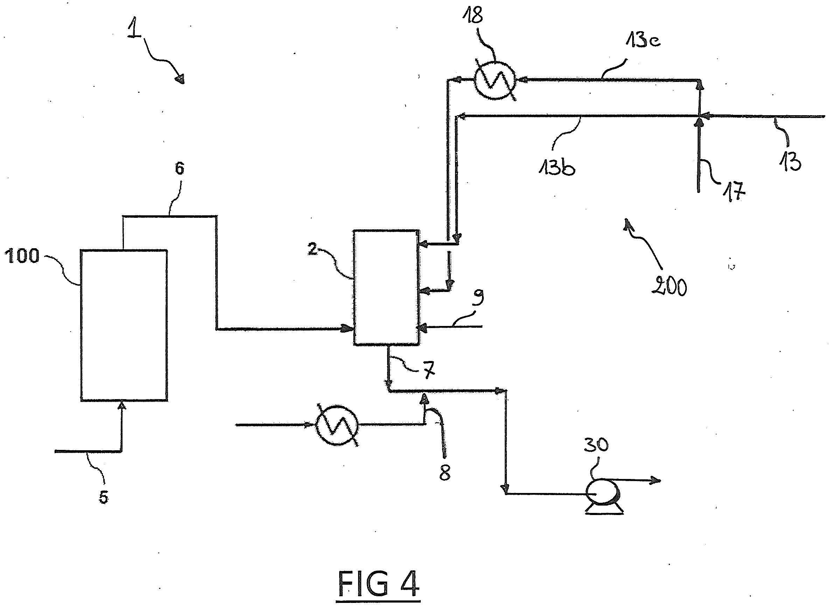

According to another embodiment of the invention, the plant of the prior art is revamped to provide the melamine plant illustrated in Fig. 4, by means of the following operations:
redirecting the stripping medium (9) to the quencher (2);
installation of a line 13 for recycling a stream of water from the plant;
installation of a line 17 for injecting ammonia into said stream of water;
installation of a line 13c for feeding a first portion of said stream of water containing ammonia to the second heat exchanger 18 and then to the quencher 2;
installation of a line 13b for feeding a second portion of said stream of water containing ammonia to the quencher 2;
installation of a line for exporting said second melamine-containing stream 7 from said quencher 2, said stream 7 being sent to a further purification section (not shown);
installation of a pump 30 downstream the quencher 2, which receives said second melamine-containing stream 7 before being sent to said further purification section;
dismissing the stripper 3, the first heat exchanger 12, the absorber 4 and the cooler 16;
discontinuing the inlet and outlet lines to / from the stripper 3, the first heat exchanger 12, the absorber 4 and the cooler 16;
discontinuing the line 30 for extracting the off-gas 30 from the quencher 2.

According to this embodiment, the revamping of the purification section 200 is carried out after revamping of the high pressure synthesis section to provide the synthesis section of Fig. 2.

## Claims

1. A high pressure melamine plant comprising a synthesis section (100) and a melamine purification section (200), wherein said synthesis section (100) provides a first melamine-containing stream (6), and said melamine purification section (200) comprises:
a quencher (2) receiving said first melamine-containing stream (6) from the synthesis section (100);
a stripper (3) fed with a second melamine-containing stream (7) from said quencher (2) and also fed with a stripping medium (9), wherein purified melamine (11) is obtained and vapours (10) are extracted;
a heat exchanger (12), wherein said vapours (10) from the stripper (3) are at least partially condensed by heat exchange with a cooling medium (13a), obtaining a condensed stream (14);
an absorber (4) fed with said condensed stream (14) from said heat exchanger (12) and providing an aqueous solution (15) comprising ammonia and carbon dioxide, at least a portion (15b) of said aqueous solution being exported from the purification section (200) of the plant.

2. Plant according to claim 1, wherein a first portion (15a) of said aqueous solution (15) is recirculated inside the absorber (4) to act as absorbing medium for said condensed stream, and a second portion (15b) of said aqueous solution (15) is exported from the purification section (200) of the plant.

3. Plant according to claim 1 or 2, comprising a line (13) arranged to introduce a stream of water into the purification section (200), a first portion (13a) of said stream of water (13) forming the cooling medium and a second portion (13b) of said stream of water (13) being directed to the quencher (2).

4. Plant according to claim 3, comprising a line (17) arranged to inject ammonia into said stream of water (13).

5. Plant according to any one of the previous claims, wherein said cooling medium (13c) leaving said heat-exchanger (12) is at least partially supplied to the quencher (2), preferably after being heated in a further heat exchanger (18).

6. Plant according to any one of the previous claims, wherein said synthesis section (100) comprises a first melamine reactor (101), a second stripping reactor (102) and a scrubber (103).

7. A process for purifying a melamine-containing stream (6) from the synthesis section (100) of a high pressure melamine plant, in a melamine purification section (200) of said plant, wherein said melamine-containing stream (6) is subjected to a quenching process which dissolves melamine to provide a second melamine-containing stream (7); said second stream (7) is subjected to stripping with a stripping medium (9) which provides purified melamine (11) and vapours (10); said vapours (10) are at least partially condensed by heat exchange with a cooling medium (13a) providing a condensed stream (14); said condensed stream (14) is subjected to an absorption process providing an aqueous solution (15), which is at least partially (15b) exported from said purification section.

8. Process according to claim 7, wherein a first portion (13a) of a stream of water (13) forms said cooling medium and a second portion (13b) of said stream of water (13) is directed to the quenching process.

9. Process according to claim 8, wherein ammonia (17) is injected into said stream of water (13).

10. Process according to any of claims 7 to 9, wherein said cooling medium (13c) used to condense the vapours (10) from the stripping process is directed to the quenching process, preferably after being further heated.

11. A method for revamping a melamine purification section (200) of a high pressure melamine plant comprising:
a quencher (2) fed with a first melamine-containing stream (23) from the synthesis section (100) and an aqueous solution (15d) comprising ammonia and carbon dioxide, wherein melamine (7) is dissolved and off-gas (30) are extracted;
a stripper (3) fed with a second melamine-containing stream (7) from said quencher (2) and with a stripping medium (9), wherein purified melamine (11) is obtained and vapours (10) are extracted, said vapours being added with a stream of water (32) once extracted from the stripper (3);
a first heat exchanger (12), wherein the vapours (10) added with said stream of water (32) are at least partially condensed by heat exchange with an aqueous solution (15d) comprising ammonia and carbon dioxide, providing a condensed stream (14);
an absorber (4) fed with said condensed stream (14) and a carbonate solution (31) and providing an aqueous solution (15) comprising ammonia and carbon dioxide, which is at least partially used for condensing the vapours (10) in said first heat exchanger (12), heated in a second heat-exchanger (18) and recycled to the quencher (2),
said method being **characterized by**
installation of a line (13a) for at least partially feeding a stream of water, possibly added with ammonia, to the first heat exchanger (12) for condensing said vapours (10);
installation of a line (13c) for at least partially sending the water leaving said heat exchanger (12) to the quencher (2);
installation of a line (15b) for at least partially exporting the aqueous solution provided by said absorber (4) from the purification section (200) of the plant;
discontinuing the line (15d) for feeding said aqueous solution to the first heat exchanger (12), for subsequent feeding to the second-exchanger (18) and recycle to the quencher (2);
discontinuing the line (32) for adding said stream of water (32) to the vapours (10) extracted from the stripper (3);
discontinuing the line (31) for introducing the carbonate solution into the absorber (4).

12. A method according to claim 11, wherein a line (13b) is installed for feeding a portion of said stream of water, possibly added with ammonia, to the quencher (2) bypassing the first heat exchanger (12).

13. A method according to claim 11 or 12, a portion (15c) of the aqueous solution extracted from the absorber (4) being directly supplied to the quencher (2), the method being **characterized by** discontinuing the line for directly supplying said aqueous solution to the quencher.

14. A method according to any one of claims 11 to 13, wherein the line (30) for extracting the off-gas (30) from the quencher (2) is discontinued.

15. A method for revamping a melamine purification section (200) of a high pressure melamine plant comprising:
a quencher (2) fed with a first melamine-containing stream (23) from the synthesis section (100) and an aqueous solution (15d) comprising ammonia and carbon dioxide, wherein melamine (7) is dissolved and off-gas (30) are extracted;
a stripper (3) fed with a second melamine-containing stream (7) from said quencher (2) and with a stripping medium (9), wherein purified melamine (11) is obtained and vapours (10) are extracted;
a first heat exchanger (12), wherein said vapours (10) are at least partially condensed by heat exchange with an aqueous solution (15d), providing a condensed stream;
an absorber (4) fed with said condensed stream from said first heat exchanger (12) and providing an aqueous solution (15), which is at least partially used for condensing the vapours (10) in said first heat exchanger (12), re-condensed in a second heat-exchanger (18) and recycled to the quencher (2),
said method being **characterized by**
installing a line (13) feeding a stream of water, possibly added with ammonia, to the quencher (2), at least a portion (13c) of said stream of water being heated inside said second heat exchanger (18);
dismissing the stripper (3);
dismissing the first heat exchanger (12);
redirecting the stripping medium (9) to the quencher (2);
installing a line for exporting said second melamine-containing stream (7) from said quencher (2);
dismissing the absorber (4).

16. A method according to any of claims 11 to 15, wherein a line (17) for injecting ammonia to said stream of water (13) is installed.
